# EUROPÄISCHE PATENTSCHRIFT

(11) **EP 1 359 903 B1**
(45) Veröffentlichungstag und Bekanntmachung des Hinweises auf die Patenterteilung: **06.04.2005**
(21) Anmeldenummer: 02711826.4
(22) Anmeldetag: 04.02.2002
(51) Int. Cl.: A61K 9/70, B65H 35/00

(54) **VERFAHREN UND VORRICHTUNG ZUR HERSTELLUNG DÜNNER PLÄTTCHEN AUS EINEM WIRKSTOFFFILM**
METHOD AND DEVICE FOR PRODUCING THIN WAFERS FROM A FILM OF ACTIVE INGREDIENTS
PROCEDE ET DISPOSITIF POUR LA PRODUCTION DE PLAQUETTES MINCES A PARTIR D'UN FILM DE PRINCIPES ACTIFS

(30) Priorität: 14.02.2001 US 268805 P; 05.03.2001 DE 10110494
(43) Veröffentlichungstag der Anmeldung: 12.11.2003
(73) Patentinhaber: LTS LOHMANN Therapie-Systeme AG, 56626 Andernach (DE)
(72) Erfinder: SCHÄFER, Wolfgang, NJ 07940 (US); HACKBARTH, Ronald, 56075 Koblenz (DE); NEULAND, Detlev, 88239 Wangen-Neuravensburg (DE)
(74) Vertreter: Flaccus, Rolf-Dieter, Dr.
(86) Internationale Anmeldenummer: PCT/EP2002/001107
(87) Internationale Veröffentlichungsnummer: WO 2002/064123

(56) Entgegenhaltungen:
- DE-A- 19 925 339
- DE-C- 949 169
- US-A- 6 106 930

## Beschreibung

Die Erfindung betrifft ein Verfahren sowie eine Vorrichtung zur Herstellung dünner Plättchen aus einem Wirkstofffilm eines oder mehrerer Wirkstoffe, insbesondere zur Verwendung als Dosier- und Darreichungsform für Arzneimittel.

Neben den bekannten Dosierformen für Arzneimittel, wie Tabletten, Kapseln, Tropfen oder ähnliche Darreichungsformen, gibt es auch die Darreichungsform des "Wafer". Es handelt sich bei diesem um ein dünnes Plättchen aus oder mit einem Wirkstofffilm mit einer vorbestimmten Wirkstoffmenge, das in seiner Dicke und seinen Abmessungen auf die abzugebende Wirkstoffmenge abgestimmt ist. Da die Kontaktfläche des Wafers im direkten Zusammenhang mit der Dosiermenge des Wirkstoffes steht, müssen seine Abmessungen weitestgehend mit den errechneten übereinstimmen und im Toleranzbereich liegen. Daher ist die Herstellung des Wafers aufwendig.

Es ist bekannt, den Wirkstoffilm mittels Gießverfahren oder durch ein Beschichtungsverfahren herzustellen. Üblicherweise wird der Wirkstofffilm mit oder ohne das Trägermaterial, auf das das Filmmaterial gegossen oder anderweitig aufgebracht wurde, in Rollen aufgewickelt und bevorratet. Wird der Wirkstofffilm mit dem Trägermaterial aufgerollt, so wird dieses bei der weiteren Verarbeitung des Wirkstofffilms zu dünnen Plättchen von diesem getrennt und separat aufgewickelt. Der dünne und flexible Wirkstofffilm wird geschnitten. Die dabei erreichten Istabmessungen entsprechen oft nicht den geforderten, der Arzneimitteldosierung entsprechenden vorgeschriebenen Maßtoleranz, die sich über die Fläche in der wirkstofftoleranz widerspiegelt.

Ein solches Verfahren ist aus US-A-6 106 930 bekannt.

Außerdem ist im technischen Gebiet der Druckmaschinen aus der Patentschrift DE-C-949169 eine Vorrichtung bekannt, die eine Halterung zum Anordnen einer Vorratsrolle mit einem Film, eine angetriebene Vorschubwalze zum Abziehen desselben von der Vorratsrolle, eine aus der Vorschubwalze und einer Schneidmessereinrichtung gebildete Längsschneidvorrichtung und eine Querschneidvorrichtung für die geschnittenen Längsstreifen aufweist, wobei zwischen der Längsschneidvorrichtung und der Querschneidvorrichtung eine Stapeleinrichtung sowie Mittel, die die geschnittenen Filmstreifen aufeinander glätten und der Querschneidvorrichtung zuführen, angeordnet sind.

Aufgabe der Erfindung ist es, ein Verfahren sowie eine Vorrichtung zur Herstellung dünner Plättchen aus einem Wirkstofffilm anzugeben, mit dem Plättchen in vorbestimmten Größen weitestgehend exakt herstellbar sind.

Diese Aufgabe wird erfindungsgemäß durch ein Verfahren gemäß Anspruch 1 und eine Vorrichtung gemäß Anspruch 6 gelöst.

Danach wird bei einem Verfahren, mit dem aus einem Wirkstofffilm dünne Plättchen hergestellt werden, nachdem dieser durch Gießen des Filmmaterials auf ein Trägermatax-ial oder Beschichten eines solchen hergestellt und mit oder ohne Trägermaterial auf einer Rolle bevorratet ist, und bei dem der Wirkstofffilm von der Vorratsrolle abgezogen und geschnitten wird, der Wirkstofffilm automatisch von der Vorratsrolle abgezogen, von einem vorhandenen Trägermaterial getrennt und gespannt einer Streifen-Schneidstation zugeführt und an dieser zunächst in Längsrichtung, die der Vorschubrichtung entspricht, in schmale Streifen einer vorbestimmten Breite geschnitten. Anschließend werden die Streifen in Vorschubrichtung zusammengeführt und als Bündel durch eine weitere Vorschubeinrichtung einem Querschneider zugeführt, der das Bündel in vorbestimmten Abständen durchschneidet. Das im räumlichen und zeitlichen Abstand aufeinanderfolgende Längs- und Querschneiden des Wirkstofffilms bringt viereckige, insbesondere rechteckige oder auch quadratische Plättchen hervor, wobei der Wirkstofffilm zumindest in seiner Breite so dimensioniert wird, daß dieser ohne Rest und Abfall in Plättchen einer vorbestimmten Größe zerschnitten wird. Dabei hat die Größe der Plättchen Einfluß auf die Dosierung. Das Zuführen des Wirkstofffilms zur Streifen-Schneidstation unter Vorspannung erleichtert den Schneidvorgang. Ebenso erleichtert und vereinfacht das Zusammenführen der in Längsrichtung geschnittenen Filmstreifen zu einem Streifenbündel das Querschneiden und erhöht überdies die Prozeßsicherheit und die Maßgenauigkeit bei der Herstellung der Plättchen.

In vorteilhafter Weise erfolgt das Abziehen des Wirkstofffilms von der Vorratsrolle und das Zuführen zur Längsschneidstation kontinuierlich, ebenso das Längsschneiden zu Filmstreifen.

Zur Erleichterung des Längsschneidens wird im Wirkstofffilm eine Vorspannung erzeugt, die zu einer Glättung desselben und damit auch zu einem exakten Längsschneidvorgang führt. Auf einfache Weise kann dazu der Wirkstofffilm quer zur Vorschubrichtung belastet werden, insbesondere durch ein definiertes Gewicht, das in Verbindung mit der Breite des Wirkstofffilms die Vorspannung bestimmt.

Die geschnittenen Filmstreifen werden bevorzugt durch Zusammenführen zu einem Stapel gebündelt, so daß diese glatt aufeinanderliegen. Das weitere Vorschieben der Filmstreifen erfolgt auf einfache Weise intermittierend und kann sicher gestaltet werden. Zum Vorschieben und Zuschieben zum Querschneider wird der Stapel durch an der Ober- und Unterseite an diesem angreifende Klemmbacken gegriffen und geklemmt und intermittierend dem Querschneider zugeschoben. Um auch in diesem Verfahrensabschnitt mit hoher Prozeßsicherheit und Maßgenauigkeit verfahren zu können, wird der Stapel vor der Klemmeinrichtung zur Sicherstellung einer exakten Übereinanderanordnung der Streifen, verbunden mit einem nochmaligen Glätten und Erzeugen einer Vorspannung, maßhaltig gleitgeführt, dabei durch die Vorschubeinrichtung gezogen und dem Querschneider zugeführt, wobei Relativbewegungen zwischen den einzelnen Streifen und unterschiedliche Material- bzw. Streifenlängungen unterbunden werden.

Eine Vorrichtung zur Durchführung des Verfahrens weist eine Halteeinrichtung für eine vorratsrolle mit dem Filmmaterial, eine elektromotorisch angetriebene Vorschubwalze zum Abziehen des Wirkstofffilms von der Vorratsrolle mindestens in der Breite desselben, eine Streifenschneideinrichtung, gebildet aus der Vorschubwalze und einer mit dieser zusammenwirkenden Schneidmessereinrichtung, eine Einrichtung zum zusammenführen und Stapeln (Stapeleinrichtung) der vorgeschobenen geschnittenen Filmstreifen und eine weitere Vorschubeinrichtung für diese sowie eine Querschneideinrichtung auf. Zwischen der Stapelvorrichtung und der weiteren Vorschubeinrichtung sind Mittel zum Glätten des Streifenstapels und in Verbindung mit der weiteren Vorschubeinrichtung Mittel zum Erzeugen einer Vorspannung angeordnet.

Im Vorschubbereich zwischen der Vorratsrolle und der Vorschubwalze sind parallel zu diesen zwei Umlenkrollen für den wirkstofffilm angeordnet, zwischen denen zur Erzeugung einer Spannung in diesem eine Tänzerrolle auf diesem aufliegt. Die Schneidmessereiarichtuag weist im Abstand nebeneinander drehbare runde Messerscheiben auf, die parallel zur Vorschubrichtung an einer Halteeinrichtung im Abstand nebeneinander gehaltert sind, die gegen den auf der Vorschubwalze vorgeschobenen wirkstofffilm gedrückt werden. Die Messerscheiben sind in ihrem Abstand zueinander einstellbar.

Bestandteil der Vorrichtung ist eine Zusammenführ- und Stapeleinrichtung für die geschnittenen vorgeschobenen Streifen. Eine derartige Vorrichtung ist in der DE 199 25 339 A1 beschrieben.

Der Vorschub der zusammenzuführenden und zu stapelnden Filmstreifen erfolgt intermittierend durch einen Zangenvorschub, der den Stapel intermittierend greift und eine Zugkraft auf diesen ausübt, und der auch den Stapel dem Querschneider zuschiebt.

Zwischen der Stapeleinrichtung und dem Zangenvorschub ist eine U-förmige Dreiseiten-Gleitbahn angeordnet, in der der Stapel durch den Zangenvorschub bewegt wird. An dieser Gleitbahn sind ortsfest, jedoch in Vertikalführungen beweglich gehaltert, Gewichte angeordnet, die auf dem bewegten Stapel aufliegen und diesen bei dessen Vorschubbewegung glätten. Die Gleitbahn ist um eine Achse quer zur Vorschubrichtung in der Draufsicht auf den Stapel konvex gewölbt, so daß in dem Stapel eine definierte Vorspannung erzeugt wird, die durch den Zangenvorschub als exakt definierte Widerstandkraft überwunden werden muß. Die Vorschubstrecke des Zangenvorschubs bestimmt die Länge der Plättchen.

Der Zangenvorschub ist mit zwei Klemmbacken versehen, die über eine Transportkurve mit einem Antrieb gekoppelt sind und, bezogen auf die Vorschubrichtung, vor und zurück bewegt werden, wobei die Klemmbacken zur Vorwärtsbewegung den Stapel im Abstand vom querschneiderseitigen Stapelende zwischen sich festgeklemmen und gegen die auf der Gleitbahn erzeugte Widerstandskraft von der Gleitbahn ziehen und dabei gleichzeitig den in Vorschubrichtung vor dem Zangenvorschub befindlichen Stapelabschnitt mit definierter Länge in den Querschneider schieben. Die Transportkurve ist dabei exzentrisch gelagert, und die Klemmbacken stehen mit dieser in ständigem Eingriff, so daß eine fortwährende Drehung der Transportkurve in eine definierte Vor- und Zurück-Translationsbewegung der Klemmbacken umgesetzt wird. Eine Veränderung der Abmessungen oder der Form der Transportkurve führt zu einer Veränderung des Größe der Translationsbewegung und damit des Vorschubs, wodurch die Abschnittslänge des Stapels und damit die Länge der Plättchen vorbestimmt werden kann.

Die Erfindung wird nachstehend anhand eines Ausführungsbeispiels erläutert. Die zugehörige Zeichnung zeigt eine Vorrichtung zur Herstellung dünner Plättchen aus einem Wirkstofffilm, teilweise schematisch.

Diese weist eine Vorratsstelle 1 mit einem Vorrat an Wirkstofffilm 2 mit einer Dicke von ca. 0,05 mm in einer nicht dargestellten Halterung, eine motorische M angetriebene Vakuumwalze 3 als Vorschubwalze zum Abziehen des Wirkstofffilms 2 von der Vorratsrolle 1, eine mit der Vakuumwalze 3 und einer mit dieser zusammenwirkenden Schaeidmessereinrichtung 4 gebildete Längsschneidvorrichtung 5, eine Stapeleinrichtung 6 für die geschnittenen Längsstreifen 7, eine im Querschnitt U-förmige Dreiseiten-Gleitbahn 8 für den gebildeten Stapel 9 aus den Längsstreifen 7, einen Zangenvorschub 10 und eine Querschneideinrichtung 11 für den Stapel 9 auf. Zwischen der Vorratsrolle 1 und der Längsschneidvorrichtung 5 sind parallel zur Vorratsrolle 1 zwei Umlenkrollen 12 für den Wirkstofffilm 2 und zwischen diesen Umlenkrollen 12 eine diesen Wirkstofffilm 2 beaufschlagende Tänzerrolle 13 zur Straffung desselben angeordnet.

Die Schneidmesservorrichtung 4 besteht aus einer nicht weiter dargestellten Halteeinrichtung 14 und an dieser nebeneinander im einstellbaren Abstand gehalterten, parallel zur Vorschubrichtung des Wirkstofffilms 2 ausgerichteten drehbaren runden Messerscheibe 15, die gegen den Wirkstofffilm 2 gedrückt sind und diesen bei dessen Vorschub durch die Vakuumwalze 3 entsprechend ihrer Anordnung längsschneiden. In der beispielhaften Ausführung beträgt der Abstand der Messerscheiben 15 20 mm.

Die Stapeleinrichtung 6 weist in einem Stapelblock 16 für jeden Längsstreifen 7 einen mit einer nicht dargestellten Vakuumeinrichtung gekoppelten Vakuum-Förderkanal 17 auf, der an einem Gemeinschaftseingang 18 mit den übrigen Vakuum-Förderkanälen 17 in Nebeneinanderanordnung angeschlossen ist. Die Kanäle 17 sind in dem Stapelblock 16 so geführt, daß sie an einem Gemeinschaftsausgang 19 in Übereinanderanordnung zusammengeführt sind.

Im Anschluß an die Stapeleinrichtung 6 folgt die Dreiseiten-Gleitbahn 8. Diese ist um eine quer zur Vorschubrichtung bzw. parallel zur Achse der Vakuumwalze 3 und der Rollen 1, 12 und 13 angeordnete Achse A gebogen und nimmt den Stapel 9 seitengeführt auf, wobei die Seitenwände der Dreiseiten-Gleitbahn 8 eine lichte Weite haben, die im wesentlichen der Breite des Stapels 9 entspricht. Auf dem Stapel 9 ist in einer nicht dargestellten Vertikalführung ein Gewicht 20 von 120 g angeordnet, das auf dem Stapel aufliegt und diesen zusammendrückt und dabei glättet.

In Vorschubrichtung ist nach der Dreiseiten-Gleitbahn 8 der Zangenvorschub 10 angeordnet, der zwei übereinander angeordnete Klemmbacken 21 und 22 zum intermittierenden Greifen des Stapels 9 aufweist, die über eine austauschbare exzentrische Transportkurve 23 mit einem Antrieb 24 gekoppelt sind. Diese (23, 24) bewegen die Klemmbacken 21 und 22, bezogen auf die Vorschubrichtung, vor und zurück, wobei die Klemmbacken 21 und 22 den Stapel 9 in ihrer hinteren Totpunktstellung I aufnehmen und in ihrer vorderen Totpunktstellung II freigeben.

Im Anschluß an den Zangenvorschub 10 sind Führungsbacken 25 angeordnet, zwischen denen der Stapel 9 zur Querschneideinrichtung 11 gleitet, in der dieser zwischen zwei Schneidmessern 26 quergeschnitten wird. Die vorbestimmbare Länge der Stapelabschnitte beträgt 25 mm, so daß sich eine Wafergröße von 20 x 25 mm ergibt.

Die hergestellten Wafer werden anschließend auf einer Verpackungsmaschine in Dispenser, Blister oder Schlauchbeutel verpackt.

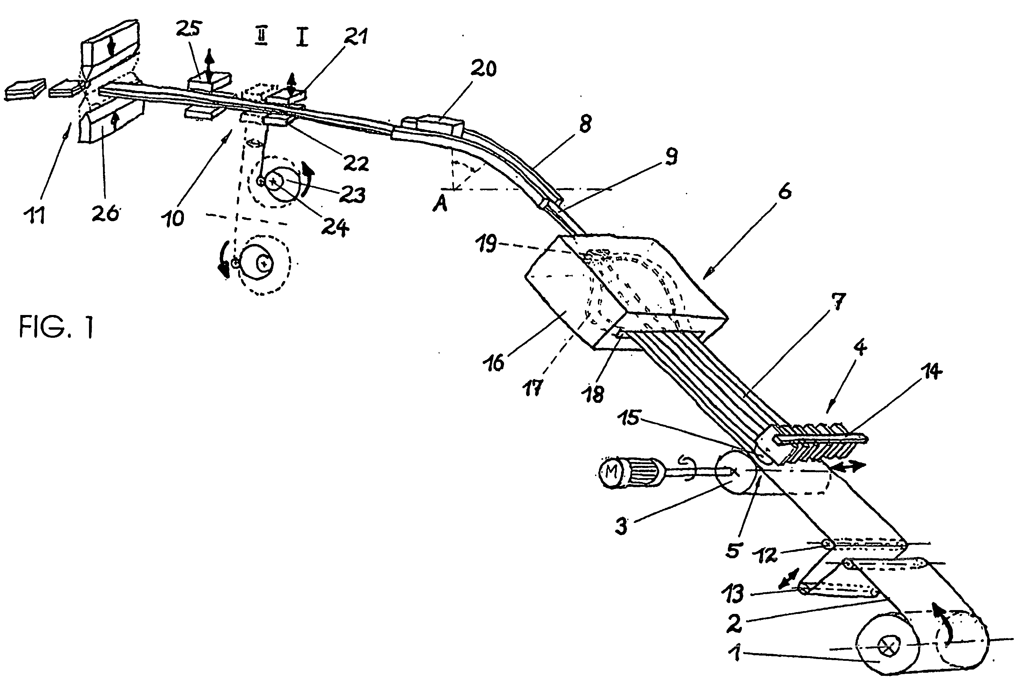

## Patentansprüche

1. Verfahren zur Herstellung dünner Plättchen aus einem Wirkstofffilm (2), bei dem dieser durch Gießen des Filmmaterials auf ein Trägermaterial oder Beschichten eines solchen erzeugt, mit oder ohne Trägermaterial auf einer Rolle (1) bevorratet, von dieser abgezogen und geschnitten wird, wobei der Wirkstofffilm (2) automatisch abgezogen, von einem vorhandenen Trägermaterial getrennt und gespannt einer Schneidstation zugeführt und in Vorschubrichtung in schmale Längsstreifen (7) einer vorbestimmbaren Breite geschnitten wird, **dadurch gekennzeichnet, daß** die Längsstreifen (7) in Vorschubrichtung zusammengeführt und gemeinsam durch eine weitere Vorschubeinrichtung einem Querschneider (11) zugeführt werden, der die zusammengeführten Längsstreifen (7) in vorbestimmten Abständen durchschneidet.

2. Verfahren nach Anspruch 1, **dadurch gekennzeichnet, daß** das Abziehen des Wirkstofffilms (2) von der Vorratsrolle (1) und das Zuführen desselben zur Schneidstation kontinuierlich durch eine angetriebene Walze erfolgt, über die der Wirkstofffilm (2) geführt und auf der dieser in Längsstreifen (7) geschnitten wird.

3. Verfahren nach Anspruch 1, **dadurch gekennzeichnet, daß** die Längsstreifen (7) zusammengeführt und als Stapel (9) übereinander angeordnet werden.

4. Verfahren nach Anspruch 1 oder 3, **dadurch gekennzeichnet, daß** der Stapel (9) durch die weitere Vorschubeinrichtung über eine Gleitführung gezogen und geglättet wird.

5. Verfahren nach Anspruch 1, **dadurch gekennzeichnet, daß** der Stapel (9) intermittierend in den Querschneider (11) eingeschoben wird.

6. Vorrichtung zur Durchführung des Verfahrens gemäß Anspruch 1, wobei diese eine Halterung zum Anordnen einer Vorratsrolle (1) mit dem Wirkstofffilm (2), eine angetriebene Vorschubwalze zum Abziehen desselben von der Vorratsrolle (1), eine aus der Vorschubwalze und einer Schneidmessereinrichtung (4) gebildete Längsschneidvorrichtung (5) und eine Querschneidvorrichtung (11) für die geschnittenen Längsstreifen (7) aufweist, wobei zwischen der Längsschneidvorrichtung (5) und der Querschneidvorrichtung (11) eine Stapeleinrichtung (16), die eine Anordnung von Förderkanälen (17) aufweist, sowie Mittel, die die geschnittenen Filmstreifen (7) glätten und der Querschneidvorrichtung (11) zuführen, angeordnet sind.

7. Vorrichtung nach Anspruch 6, **dadurch gekennzeichnet, daß** zwischen der Vorratsrolle (1) und der Längsschneidvorrichtung (5) parallel zur Vorratsrolle (1) zwei Umlenkrollen (12) für den Wirkstofffilm (2) angeordnet sind, zwischen denen dieses durch eine Tänzerrolle (13) beaufschlagt ist.

8. Vorrichtung nach Anspruch 7, **dadurch gekennzeichnet, daß** die Schneidmessereinrichtung (4) aus einer Halteeinrichtung (14) und an dieser nebeneinander im einstellbaren Abstand gehalterten, parallel zur Vorschubrichtung des Wirkstofffilms (2) ausgerichteten drehbaren runden Messerscheiben (15) gebildet ist.

9. Vorrichtung nach Anspruch 6, **dadurch gekennzeichnet, daß** die Mittel zum Stapeln aus einer Anordnung von Vakuum-Förderkanälen (17) bestehen, deren Eingänge (18) hebeneinander und deren Ausgänge (19) übereinander angeordnet sind, wobei jeweils einem Längsstreifen (7) ein Vakuum-Förderkanal (17) zugeordnet ist.

10. Vorrichtung nach Anspruch 9, **dadurch gekennzeichnet, daß** nach der Stapeleinrichtung eine im Querschnitt U-förmige Dreiseiten-Gleitbahn (8) für den gebildeten Stapel (9) angeordnet ist, an der ortsfest in Vertikalführungen wenigstens ein Gewicht (20) gehaltert ist, das auf dem auf der Gleitbahn (8) bewegten Stapel (9) gleitet und die Längsstreifen (7) glättet.

11. Vorrichtung nach Anspruch 10, **dadurch gekennzeichnet, daß** die Dreiseiten-Gleitbahn (8) um eine Achse quer zur Vorschubrichtung nach unten gewölbt ist.

12. Vorrichtung nach Anspruch 10 oder 11, **dadurch gekennzeichnet, daß** in Vorschubrichtung nach der Dreiseiten-Gleitbahn (8) ein Zangenvorschub (10) angeordnet ist, der den Stapel (9) intermittierend greift und der Quersehneidvorrichtung (11) zuführt.

13. Vorrichtung nach Anspruch 12, **dadurch gekennzeichnet, daß** der Zangenvorschub (10) zwei Klemmbacken (21,22) für den Stapel (9) aufweist, die über eine austauschbare exzentrische Transportkurve (23) mit einem Antrieb (24) gekoppelt sind, der die Klemmbakken (21,22), bezogen auf die Vorschubrichtung, vor und zurück bewegt, und daß die Klemmbacken den Stapel (9) bei ihrer Vorwärtsbewegung festklemmen.

14. Vorrichtung nach Anspruch 13, **dadurch gekennzeichnet, daß** zwischen dem Zangenvorschub (10) und der Querschneidvorrichtung (11) Führungselemente (25) angeordnet sind.

## Claims

1. Process for producing small, thin sheets from an active-ingredient film (2), in which the latter is produced by casting the film material onto a substrate material or by coating a substrate material, storing it with or without substrate material on a reel (1), pulling it off the reel (1) and cutting it, wherein the active-ingredient film (2) is pulled off automatically, is separated from a substrate material which is present and in tensioned form is fed to a cutting station and is cut, in the feed direction, into long, narrow strips (7) of predeterminable width, **characterized in that** the long strips (7) are brought together in the feed direction and together are fed by a further feed device to a transverse cutter (11), which cuts through the combined long strips (7) at predetermined intervals.

2. Process according to claim 1, **characterized in that** the active-ingredient film (2) is pulled off the storage reel (1) and is fed continuously to the cutting station by means of a driven roller, over which the active-ingredient film (2) is guided and on which this film is cut into long strips (7).

3. Process according to claim 1, **characterized in that** the long strips (7) are brought together and are arranged on top of one another in the form of a stack (9).

4. Process according to claim 1 or 3, **characterized in that** the stack (9) is pulled over a sliding guide by the further feed device and is smoothed.

5. Process according to claim 1, **characterized in that** the stack (9) is intermittently pushed into the transverse cutter (11).

6. Device for carrying out the process according to claim 1, wherein this device has a holding means for positioning a storage reel (1) with the active-ingredient film (2), a driven feed roller for pulling the film (2) off the storage reel (1), a longitudinal cutting device (5), which is formed from the feed roller and a cutting-blade device (4), and a transverse cutting device (11) for the long cut strips (7), wherein a stacking device (16), provided with an arrangement of conveyor channels (17), as well as means which smooth the cut film strips (7) and feed them to the transverse cutting device (11) being arranged between the longitudinal cutting device (5) and the transverse cutting device (11).

7. Device according to claim 6, **characterized in that** two guide rollers (12) for the active-ingredient film (2) are arranged between the storage reel (1) and the longitudinal cutting device (5), parallel to the storage reel (1), between which guide rollers (12) the film is acted on by a dancer roller (13).

8. Device according to claim 7, **characterized in that** a cutting-blade device (4) is formed from a holding device (14) and rotatable round cutting discs (15) which are held next to one another on the holding device (14) at an adjustable distance from one another and are oriented parallel to the feed direction of the active-ingredient film (2).

9. Device according to claim 6, **characterized in that** the means for stacking comprise an arrangement of vacuum conveyor channels (17), the entries (18) to which are arranged next to one another and the exits (19) from which are arranged above one another, wherein each long strip (7) having an associated vacuum conveyor channel (17).

10. Device according to claim 9, **characterized in that** a three-sided slideway (8), which is U-shaped in cross section, for the stack (9) which is formed is arranged downstream of the stacking device, on which slideway (8) at least one weight (20) is held in a stationary position in vertical guides, which weight (20) slides over the stack (9) moving along the slideway (8), thus smoothing the long strips (7).

11. Device according to claim 10, **characterized in that** the three-sided slideway (8) is curved downward about an axis which lies transversely with respect to the feed direction.

12. Device according to claim 10 or 11, **characterized in that** a gripper feed system (10), which intermittently grips the stack (9) and feeds it to the transverse cutting device (11), is arranged downstream of the three-sided slideway (8), as seen in the feed direction.

13. Device according to claim 12, **characterized in that** the gripper feed system (10) has two clamping jaws (21, 22) for the stack (9), which are coupled to a drive (24) by means of an exchangeable eccentric conveyor cam (23) which moves the clamping jaws (21, 22) backward and forward with respect to the feed direction, and that the clamping jaws clamp the stack (9) during its forward movement.

14. Device according to claim 13, **characterized in that** guide elements (25) are arranged between the gripper feed system (10) and the transverse cutting device (11).

## Revendications

1. Procédé pour la production de plaquettes minces, à partir d'un film de principes actifs (2), dans lequel ce dernier est créé par coulage du matériau constituant le film sur un matériau support ou par un revêtement de ce dernier, puis stocké avec ou sans matériau support sur un rouleau (1), déroulé de ce dernier et coupé, le film de principes actifs (2) étant déroulé automatiquement, séparé d'un matériau support existant et amené à l'état tendu à un poste de coupe, puis coupé en bandes longitudinales étroites (7) dans le sens d'avance d'une largeur prédéterminable, **caractérisé en ce que** les bandes longitudinales (7) sont rassemblées dans le sens d'avance et amenées ensemble par un autre système d'avance à une machine à découper transversale (11), qui sectionne les bandes longitudinales (7) rassemblées à des intervalles prédéfinis.

2. Procédé selon la revendication 1, **caractérisé en ce que** le retrait du film de principes actifs (2) du rouleau de stockage (1) et l'acheminement de ce dernier vers le poste de coupe sont assurés de façon continue par un cylindre entraîné, par l'intermédiaire duquel le film de principes actifs (2) est guidé et sur lequel il est coupé en bandes longitudinales (7).

3. Procédé selon la revendication 1, **caractérisé en ce que** les bandes longitudinales (7) sont rassemblées et disposées en pile (9) les unes sur les autres.

4. Procédé selon la revendication 1 ou 3, **caractérisé en ce que** la pile (9) est tirée par l'autre système d'avance sur une coulisse et lissée.

5. Procédé selon la revendication 1, **caractérisé en ce que** la pile (9) est insérée par intermittence dans la machine à découper transversale (11).

6. Dispositif pour la réalisation du procédé selon la revendication 1, ce dernier présentant une fixation destinée à recevoir un rouleau de stockage (1) muni du film de principes actifs (2), un cylindre d'avance entraîné, pour retirer celui-ci du rouleau de stockage (1), un dispositif de coupe longitudinale (5) formé par le cylindre d'avance et par un système de lames (4) et un dispositif de coupe transversale (11) pour les bandes longitudinales (7) coupées, un système d'empilage (16) muni d'un agencement de canaux de transport (17) et des moyens pour lisser les bandes de film coupées (7) et les amener au dispositif de coupe transversale (11) étant disposés entre le dispositif de coupe longitudinale (5) et le dispositif de coupe transversale (11).

7. Dispositif selon la revendication 6, **caractérisé en ce que** deux rouleaux de renvoi (12) pour le film de principes actifs (2), entre lesquels ce dernier est soumis à un rouleau danseur (13), sont disposés entre le rouleau de stockage (1) et le dispositif de coupe longitudinale (5), parallèlement au rouleau de stockage (1).

8. Dispositif selon la revendication 7, **caractérisé en ce que** le système de lames (4) est formé par un système de maintien (14) et par des disques à couteaux (15) ronds rotatifs fixés sur ce dernier, les uns à côté des autres à une distance réglable, orientés parallèlement au sens d'avance du film de principes actifs (2)

9. Dispositif selon la revendication 6, **caractérisé en ce que** les moyens d'empilage sont constitués d'un agencement de canaux de transport sous vide (17), dont les entrées (18) sont disposées les unes à côté des autres et dont les sorties (19) sont disposées les unes au-dessus des autres, un canal de transport sous vide (17) étant respectivement associé à une bande longitudinale (7).

10. Dispositif selon la revendication 9, **caractérisé en ce qu'**une glissière à trois faces (8) avec une section transversale en forme de U pour la pile formée (9) est disposée en aval du système d'empilage, pile formée sur laquelle au moins un poids (20) qui glisse sur pile (9) déplacée sur la glissière (8) et qui lisse les bandes longitudinales (7) est disposé de façon fixe dans des guidages verticaux.

11. Dispositif selon la revendication 10, **caractérisé en ce que** la glissière à trois faces (8) est voûtée vers le bas autour d'un axe, transversalement au sens d'avance.

12. Dispositif selon la revendication 10 ou 11, **caractérisé en ce qu'**un système d'avance à pinces (10), qui agrippe la pile (9) de façon intermittente et l'amène au dispositif de coupe transversale (11) est disposé dans le sens d' avance, en aval de la glissière à trois faces (8).

13. Dispositif selon la revendication 12, **caractérisé en ce que** le système d'avance à pinces (10) est muni de deux mâchoires de serrage (21, 22) pour la pile (9) qui sont couplées à un entraînement (24) qui déplace les mâchoires de serrage (21, 22) vers l'avant et vers l'arrière par rapport au sens d'avance, par l'intermédiaire d'une courbe de transport excentrique interchangeable (23) et **en ce que** les mâchoires de serrage enserrent la pile (9) lors de leur déplacement vers l'avant.

14. Dispositif selon la revendication 13, **caractérisé en ce que** des éléments de guidage (25) sont disposés entre le système d'avance à pinces (10) et le dispositif de coupe transversale (11).
